# EUROPEAN PATENT SPECIFICATION

(11) **EP 1 032 429 B1**
(45) Date of publication and mention of the grant of the patent: **28.05.2003**
(21) Application number: 98962403.6
(22) Date of filing: 20.11.1998
(51) Int. Cl.: A61K 48/00

(54) **A METHOD FOR IN VIVO DNA DELIVERY USING A NEEDLE FREE APPARATUS**
VERFAHREN ZUR IN VIVO DNS-VERABREICHUNG DURCH VERWENDUNG EINES NADELFREIEN GERÄTS
PROCEDE SERVANT A ADMINISTRER DE L'ADN IN VIVO AU MOYEN D'UN DISPOSITIF SANS AIGUILLE

(30) Priority: 20.11.1997 EP 97120384
(43) Date of publication of application: 06.09.2000
(73) Proprietor: Aventis Pasteur, 69367 Lyon Cedex 07 (FR)
(72) Inventor: ERDILE, Lorne, Loudonville, NY 12211 (US); HAENSLER, Jean, F-69290 Saint Genis les Ollières (FR)
(74) Representative: Ayroles, Marie-Pauline
(86) International application number: EP9807812
(87) International publication number: WO99026662

(56) References cited:
- WO-A-94/24263
- FR-A- 2 641 190
- FURTH ET AL: "GENE TRANSFER INTO MAMMALIAN CELLS BY JET INJECTION" HYBRIDOMA, vol. 14, no. 2, 1995, pages 149-152, XP002064168 cited in the application
- FELTQUATE ET AL: "DIFFERENT T HELPER CELL TYPES AND ANTIBODY ISOTYPES GENERATED BY SALINE AND GENE GUN DNA IMMUNIZATION" THE JOURNAL OF IMMUNOLOGY, vol. 158, March 1997, pages 2278-2284, XP002098283 cited in the application
- BRANDSMA ET AL: "USE OF A RAPID, EFFICIENT INOCULATION METHOD TO INDUCE PAPILLOMAS BY COTTONTAIL RABBIT PAPILLOMAVIRUS DNA SHOWS THAT THE E7 GENE IS REQUIRED" PROCEEDINGS OF THE NATIONAL ACADEMY OF SCIENCES,USA, vol. 88, 1991, pages 4816-4820, XP002064169
- DU CHATELET ET AL: "CLINICAL IMMUNOGENICITY AND TOLERANCE STUDIES OF LIQUID VACCINE DELIVERED BY JET-INJECTOR AND A NEW SINGLE-USE CARTRIDGE (IMULE): COMPARISON WITH STANDARD SYRINGE INJECTION" VACCINE, vol. 15, no. 4, March 1997, pages 449-458, XP002064171 cited in the application
- VAHLSING ET AL: "IMMUNIZATION WITH PLASMID DNA USING A PNEUMATIC GUN" JOURNAL OF IMMUNOLOGICAL METHODS, vol. 175, 1994, pages 11-22, XP002064172 cited in the application
- ROTH ET AL: "P53 AS A TARGET FOR CANCER VACCINES: RECOMBINANT CANARYPOX VIRUS VECTORS EXPRESSING P53 PROTECT MICE AGAINST LETHAL TUMOR CELL CHALLENGE" PROCEEDINGS OF THE NATIONAL ACADEMY OF SCIENCES,USA, vol. 93, May 1996, pages 4781-4786, XP002064170 cited in the application
- HURPIN C ET AL: "The mode of presentation and route of administration are critical for the induction of immune responses to p53 and antitumor immunity." VACCINE, (1998 JAN-FEB) 16 (2-3) 208-15. JOURNAL CODE: X6O. ISSN: 0264-410X., XP002098284 ENGLAND: United Kingdom

## Description

The invention relates to a method for delivering a DNA molecule *in vivo.* Such a method has various applications *e.g.*, vaccination, cancer immunotherapy and gene therapy.

It is now well-established that non-infectious DNA molecules such as plasmids, can be delivered *in vivo* to vertebrate *e*.*g*., mammalian tissues and/or organs and as a consequence of this, the proteins encoded by the sequences operatively linked to a suitable promoter that are present in these DNA molecules are expressed either locally or elsewhere in the body of the mammal. Delivery can be achieved using a variety of administration routes and the choice depends upon the goal that is pursued ; indeed *in vivo* DNA delivery has broad applications in several fields.

More particularly, it is also known that a vertebrate *e*.*g*., a mammal can develop an immune response to an antigenic protein expressed upon DNA delivery and that the immune response can be protective. In order to induce an immune response to a particular antigen, a DNA molecule encoding this antigen is typically administered into the skin or muscles as described in US patent N° 5,580,859. The choice of the administration route depends upon a number of criteria, including *e.g.*, the type of immune response that is looked for. Indeed, the immune response may not necessarily be identical by either of the intramuscular, intraepidermal and intradermal routes. Further, some immunogens can induce a protective immune response by one route only. DNA delivery into the skin or muscles can also be used in gene therapy, so that a therapeutic protein be expressed in these tissues.

Administration can be achieved using either a syringe or a needle-free apparatus. Needle-free apparatuses are known in the art. Some of them are designed to target the sub-cutaneous tissue layer of the skin with a liquid vaccinal dose *e*.*g*., the Imojet™/Imule™ injector system (Pasteur Merieux Connaught) the use of which is described for example in Parent du Châtelet *et al* Vaccine (1997) 15 : 449. Others are useful to target the human skin *e*.*g*., the Mesoflash™ injector described in FR 2 641 190 and manufactured for use in mesotherapy by PROLITEC, rue Gustave Gresse 26400 Aouste sur Sye, France (e-mail : PROLITEC@Compuserve.com). In 1998, the trade name « Mesoflash » was changed for Isajet™.

Jet-injectors such as the Ped-O-Jet™ (Furth *et al*, Anal. Biochem. (1992) 205 : 365), a low force dermojet (Furth *et al*, Hybridoma (1995) 14 : 149) and the Med-E-Jet™ (Vahlsing *et al*, J. Immunol. Meth. (1994) 174 : 11) have successfully been used in several gene transfer assays, including a DNA immunization experiment (Vahlsing *et al*). However, none of these studies aimed at using such devices for administration of DNA mainly to the skin. Vahlsing *et al* reports on intramuscular administration, whereas Furth *et al* mentions combined skin and muscles injections.

Up to now, non-infectious DNA vaccines have been delivered to the skin using either (i) an injection device (called "Gene gun") that is adapted to propell directly into the cells of the derm or epiderm, a powder containing DNA coated on gold or tungstene microparticles (such a device is described in US patents N° 4,945,050 and 5,015,580 and WO 94/24263); or (ii) a syringe filled with a DNA solution. The main rationale for the Gene-gun, which bombards cells with DNA-coated gold microparticles, is that it overcomes membrane barriers and enables direct intracellular delivery of the DNA into skin cells including Langerhan's cells which are potent APCs (Condon *et al*, Nature Med. (1996) 2 : 1122). The Gene-gun has been considered until now to have superior delivery capabilities and to achieve appropriate intradermal immunization. Obtaining similar results with a less sophisticated injection of DNA in solution is very surprising, since plasma membranes constitute tight barriers for extracellularly delivered DNA molecules.

Indeed, it has now been found that a non-infectious DNA solution can also be delivered efficiently to the skin using a needle-free device. This method solves the drawbacks associated with the use of a syringe and is particularly suitable for *e.g.*, large scale DNA vaccination campaigns. Therefore, the invention provides for:

The according to claim 1 use of a solution comprising a non-infectious DNA molecule able to express in a vertebrate *e.g.*, a mammal, a physiologically active protein, for the manufacture of a medicament intended to be delivered exclusively to the skin of the vertebrate using a needle-free apparatus adapted to this effect;

By "non-infectious DNA molecule " is meant a DNA molecule unable to replicate in vertebrates *e.g.,* mammals and/or unable to integrate in the vertebrate *e.g.,* mammalian genome. Preferably, the DNA molecule is a plasmid. Typically, the DNA molecule comprises a sequence encoding the desired protein (polypeptide is another word for protein) that is operatively linked to a promoter (placed under the control of a promoter) suitable for expression in a vertebrate *e.g.*, a mammalian cell. The promoter can function ubiquitously or be specific for the intradermal cells. Examples of non-tissue specific promoters include the early Cytomegalovirus (CMV) promoter (described in US patent N° 4,168,062) and the Rous Sarcoma Virus promoter (described in Norton & Coffin, Mol. Cell Biol. (1985) 5 : 281). More generally, useful vectors are described in *i.a.*, WO 94/21797 and Hartikka *et al*, Human Gene Therapy (1996) 7 : 1205. Promoters specific for the cells of the skin include the keratin promoter (Lersch *et al*, Mol. Cell Biol. (1989) 9 : 3685 and Leask *et al*, Genes Dev. 4 : 1985) and the promoter CD11c (Lopez-Cabrera *et al*, J. Biol. Chem. (1993) 268 : 1187), this latter being specific for dendritic cells *i*.*a*., Langerhan's cells of the skin.

Standard techniques of molecular biology for preparing and purifying DNA can be used in the preparation of DNA molecules for use in the invention. In the soluble preparation, DNA molecules can be formulated according to various methods.

First, a polynucleotide can be used in a naked form, free of any delivery vehicles, such as anionic liposomes, cationic lipids, microparticles, *e.g.*, gold microparticles, precipitating agents, *e.g.*, calcium phosphate, or any other transfection-facilitating agent. In this case, the polynucleotide can be simply diluted in a physiologically acceptable solution, such as sterile saline or sterile buffered saline, which is preferably is isotonic, weakly hypertonic. or hypertonic and has a relatively low ionic strength, such as provided by a sucrose solution, *e.g.*, a solution containing 20 % sucrose.

Alternatively, a polynucleotide can be associated with agents that assist in cellular uptake. It can be, *i*.*a*., encapsulated into liposomes, or associated with cationic lipids. Anionic and neutral liposomes are well-known in the art (see, *e*.*g*., Liposomes: A Practical Approach, RPC New Ed, IRL press (1990), for a detailed description of methods for making liposomes) and are useful for delivering a large range of products, including polynucleotides. Cationic lipids are also known in the art and are commonly used for gene delivery. Such lipids include DOTMA (N-[1-(2,3-dioleyloxy)propyl]-N,N,N-trimethyl ammonium chloride), DOTAP (1,2-bis(oleyloxy)-3-(trimethylammonio)propane), DDAB (dimethyldioctadecylammonium bromide), DOGS (dioctadecylamidologlycyl spermine) and cholesterol derivatives such as DC-Chol (3 beta-(N-(N',N'-dimethyl aminomethane)-carbamoyl) cholesterol). A description of these cationic lipids can be found in EP 187,702, WO 90/11092, U.S. Patent No. 5,283,185, WO 91/15501, WO 95/26356, and U.S. Patent No. 5,527,928. Cationic lipids for gene delivery are preferably used in association with a neutral lipid such as DOPE (dioleyl phosphatidylethanolamine) as for example, described in WO 90/11092. For example, the commercial transfecting reagent Lipofectin™ is a DOTMA/DOPE mixture.

Other transfection-facilitating compounds can be added to a formulation containing cationic liposomes. A number of them are described in *e.g.,* WO 93/18759, WO 93/19768, WO 94/25608, and WO95/2397. They include, *i*.*a*., spermine derivatives useful for facilitating the transport of DNA through the nuclear membrane (see, for example, WO 93/18759) and membrane-permeabilizing compounds such as GALA, Gramicidine S, and cationic bile salts (see, for example, WO 93/19768). Additionally, cationic polymers and polycations constitute a large and important class of transfecting agents that can be used in the present invention.

The physiologically active protein, the *in vivo* expression of which is looked for, can be a mammalian protein able to treat a genetic defect *e.g.*, factor XIII or IX ; or a cytokine. The protein can also be an antigen that is (a) specific for a microorganism able to induce an infection in a mammal or (b) tumor-associated, with the intent to treat or prevent an infection or a tumoral disorder. The microorganism can be *e.g.*, a virus. a bacteria, a fungus or a parasite.

An example of tumor-associated antigen is the product of the p53 tumor suppressor gene. It is an attractive antigen for cancer immunotherapy, since it is expressed at very low levels in normal cells, but is overexpressed in about 50 % of all human cancers due to missense mutations that significantly increase the half-life of the protein. It has now been found that immunization of mice with a plasmid encoding human wild-type p53 induces complete protection against a challenge with a mouse fibroblast transfected with human mutant p53 and partial, but significant, protection against a transfected mastocytoma. More generally, the invention contemplates the use of a DNA molecule encoding p53, wild-type or mutant, full-length or fragments, and preferably of human origin.

The amount of DNA per dose to be administered to a recipient depends, *e*.*g*., on the strength of the promoter used in the DNA construct, the biological activity of the expressed gene product, the condition of the mammal intended for administration (*e.g.*, the weight, age, and general health of the mammal), the mode of administration, and the type of formulation. In general, a therapeutically or prophylactically effective dose from about 10 µg to about 1 mg, preferably, from about 100 µg to about 1 mg and, more preferably. from about 250 µg to about 800 µg, can be administered to human adults. The administration can be achieved in a single dose or repeated at intervals. The DNA must be administered to any of the skin layers, including the epidermis and dermis. Administration is achieved strictly by the intraepidermal or intradermal routes, traces or even no DNA at all being delivered outside the skin (*e*.*g*., in the fat, muscles or sub-cutaneous layer).

For use in the present invention, a needleless injector device may be *i*.*a*., any of those already available provided that they adjusted to administer a solution essentially into the skin. Preferably, it does not scrape the skin and the solution is propelled into the skin thanks to a source of mechanical energy *e.g.*, a gas or a spring. For example, the Mesoflash™ M40 (now called ISA40™) apparatus can be used for intradermal administration to primates *i*.*e*., monkeys and humans, while the M10 (now called ISA10™) model shall be used on mice or small vertebrate animals. In needle-free injectors, a pressure strength usually acts as means for propelling the solution. In order to target the skin, the pressure strength can be adjusted in a variety of ways, depending on the particular features of the device that is used.

It has also been found that changing the route and mode of administration of DNA vaccines can affect the nature of the induced immune response. Indeed it has been reported that antibodies *i*.*a*., anti-haemaglutinin antibodies induced by DNA injected by the intramuscular route (IM) were predominantly of the IgG2a isotype, with relatively lower levels of IgG1 antibodies, suggesting that a Th1 helper T-cell response was generated (Deck *et al*, Vaccine (1997) 15 : 71). These results are now confirmed and it is further indicated that intradermal (ID) jet immunization induces higher IgG1 titers and thus a more balanced as compared to IM immunization. When the administration of the HA-DNA vaccine to the skin is performed by using a gene gun, the antibody induced are mainly of the IgG1 subtype and the isotype balance largely in favour of a predominant Th2 helper T-cell response (Feltquate *et al*, J. Immunol. (1997) 158 : 2278). Accordingly, the present invention is particularly suitable when a balanced Th1/Th2 helper type of immune response is sought.

The Th1 response relative to the Th2 response may be estimated by comparing, for example, the IgG2a and IgG1 levels induced in mice, which are respectively indicative of the coming into play of the Th1 and Th2 responses. Indeed, the Th1 response which is sought is generally accompanied by a Th2 response. However, it is considered that the Th2 response should not be significantly predominant relative to the Th1 response. The IgG2a and IgG1 levels induced in mice can be assessed conventionally using an ELISA test, provided that the tests used for each of the two subisotypes are of the same sensitivity and, in particular, that the anti-IgG2a and anti-IgG1 antibodies are of the same affinity.

The quantities of IgG2a and IgG1 may be measured in particular using an ELISA test which is identical or similar to that described below. The wells of a polycarbonate ELISA plate are coated with 100 µl of an appropriate antigen, at about 10 µg/ml in carbonate buffer. The ELISA plate is incubated for 2 hours at 37°C and then overnight at 4°C. The plate is washed with PBS buffer (phosphate buffer saline) containing 0.05% Tween 20 (PBS/Tween buffer). The wells are saturated with 250 µl of PBS containing 1% bovine serum albumin in order to prevent nonspecific binding of the antibodies. After incubating for one hour at 37°C, the plate is washed with PBS/Tween buffer. The antiserum collected from mice, a number of days after the latter have received the composition intended to induce a Th1-type immune response, is serially diluted in PBS/Tween buffer. 100 µl of the dilutions are added to the wells. The plate is incubated for 90 minutes at 37°C, washed and evaluated according to standard procedures. For example, a goat antibody to mouse IgG2a or IgG1, coupled to an enzyme such as peroxidase, is used. The incubation in the presence of this antibody is continued for 90 minutes at 37°C. The plate is washed and then the reaction is developed with the appropriate substrate (for example O-phenyldiamine dihydrochloride when the enzyme used is peroxidase). The reaction is evaluated by colorimetry (by measuring the absorbance by spectrophotometry). The IgG2a or IgG1 titre of the antiserum corresponds to the reciprocal of the dilution giving an absorbance of 1.5 at 490 nm.

The induction of a useful Th1 response for the purposes of the present invention is marked by a ratio of the ELISA IgG2a:IgG1 titres in mice which should be around 1. When this ratio is around 1, the Th1/Th2 response is said to be mixed or balanced. When the ratio is greater than or equal to 5, the Th1 response is then said to be preponderant.

The production of a Th1 (or Th2) response in mice is predictive of a Th1 (or Th2) response in man. Although it is easier to evaluate the type of response in mice, it can also be done in man by measuring the levels of cytokines specific for the Th1 response on the one hand and, on the other hand, for the Th2 response, which are subsequently induced. The Th1 and Th2 responses can be evaluated directly in man relative to each other on the basis of the levels of cytokines specific for the two types of response on the basis of the IFN-γ/IL-4 ratio.

The invention is illustrated hereinafter by reference to the following figures :

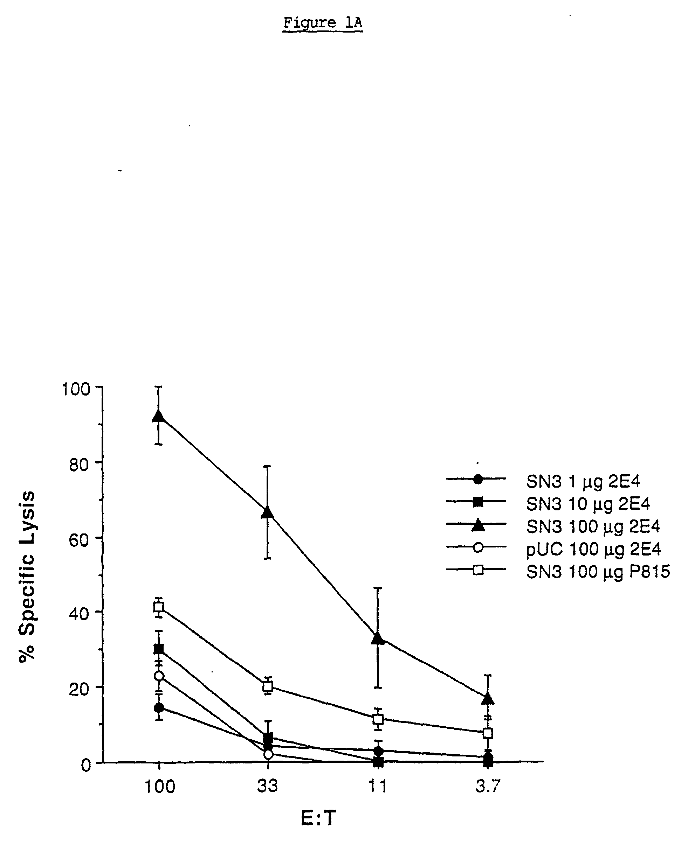

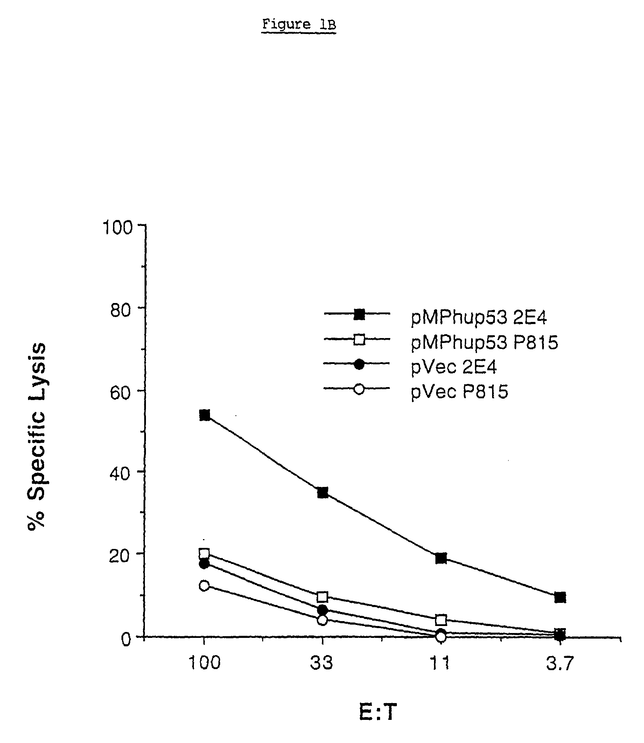

Figures 1A and 1B show the anti-p53 CTL response induced by intradermal administration of DNA. Mice were immunized intradermally at 0, 3 and 6 weeks with pC53-SN₃, or the control plasmid, pUCneo (A), or intramuscularly at 0, 3 and 6 weeks with pMP1406hup53, or vector lacking the p53 gene, pVec. The anti-p53 CTL response, 2 weeks after the last immunization, was measured in splenocytes stimulated twice *in vitro* with naïve splenocytes infected with vCP207 (A), or stimulated once *in vitro* with naive splenocytes infected with vP1101 (B). Lysis was measured against P815 and 2E4. Values in (A) are the mean of two groups of two mice with standard deviations represented by the error bars, whereas those in (B) are from a single pool of two mice.
In Figure 1A : -●- corresponds to pC53-SN₃ 1 µg / 2E4 ; -■- corresponds to pC53-SN₃ 10 µg / 2E4 ; -▲- corresponds to pC53-SN₃ 100 µg / 2E4 ; -○- corresponds to pUC 100 µg / 2E4 ; and -□- corresponds to pC53-SN₃ 100 µg / P815.
In Figure 1B : -■- corresponds to pMPhup53 / 2E4 ; -□- corresponds to pMPhup53/P815 ; -●- corresponds to pVec / 2E4 ; and -○- corresponds to pVec / P815.
Figures 2A to 2D show protection against challenge following immunization with pCP207 or pC53-SN₃. Mice (nine *per* group) were immunized at 0, 3 and 6 weeks with 10⁷ TCID50 of vCP207 or ALVAC intravenously (A, B and D), or 100 µg of pC53-SN₃ or pUCneo intradermally (C and D) or with PBS. Mice were challenged subcutaneously 2 weeks after the last injection with 10⁷ cells of clone 2-21 (A and C), 10⁷ cells of untransfected BALB/3T12-3 (B) or 10⁴ cells of clone 4D5 (D). BALB/c mice were used in (A, B and C) and DBA/2 mice in (D).

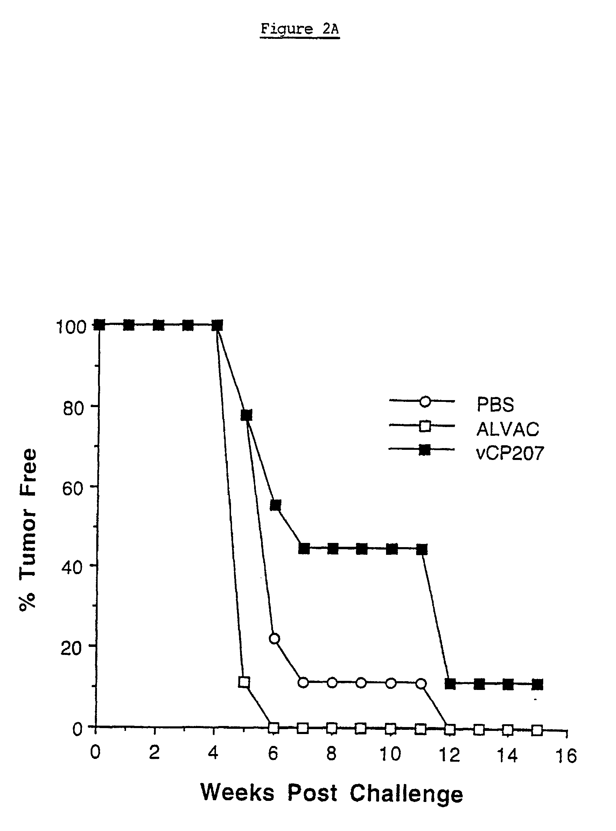

In Figure 2A : -○- corresponds to phosphate buffer saline (PBS); -□- corresponds to ALVAC ; and -■- corresponds to vCP207.

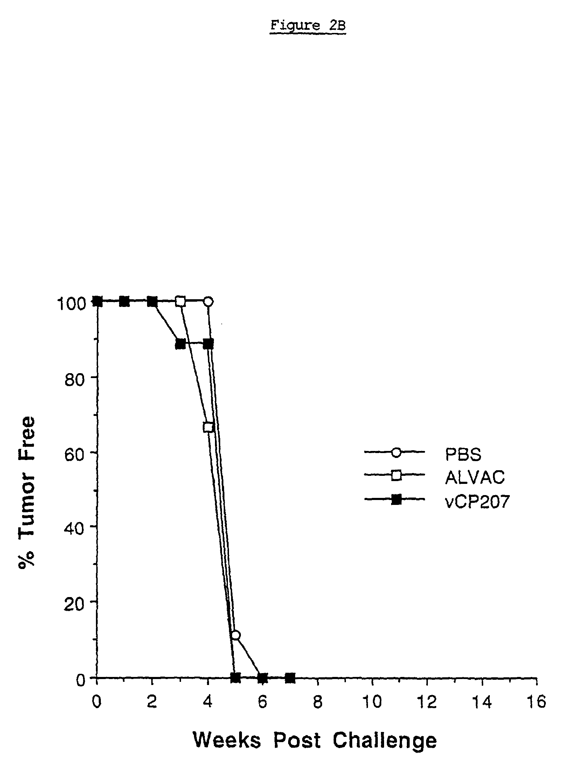

In Figure 2B : -○- corresponds to PBS ; -□- corresponds to ALVAC ; and -■- corresponds to vCP207.

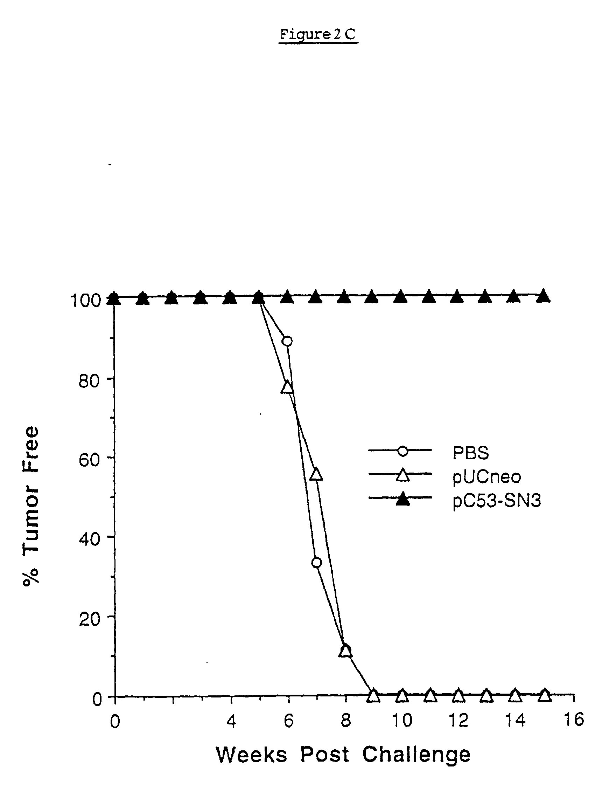

In Figure 2C : -○- corresponds to PBS ; -Δ- corresponds to PBS ; corresponds to pUCneo; and -▲- corresponds to pC53-SN₃.

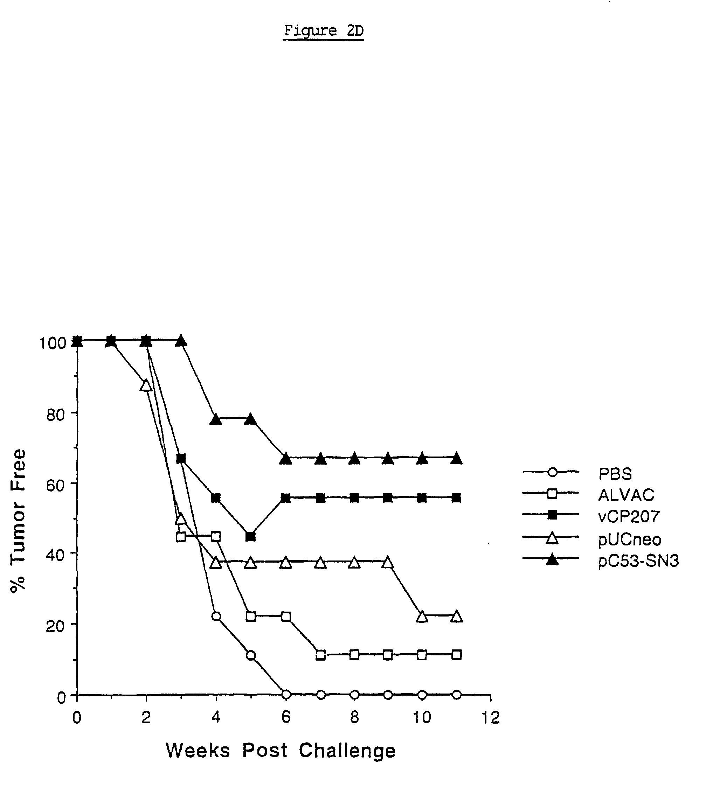

In Figure 2D : -○- corresponds to PBS ; -□- corresponds to ALVAC ; -■- corresponds to vCP207 ; -Δ- corresponds to pUCneo ; and -▲- corresponds to pC53-SN₃.

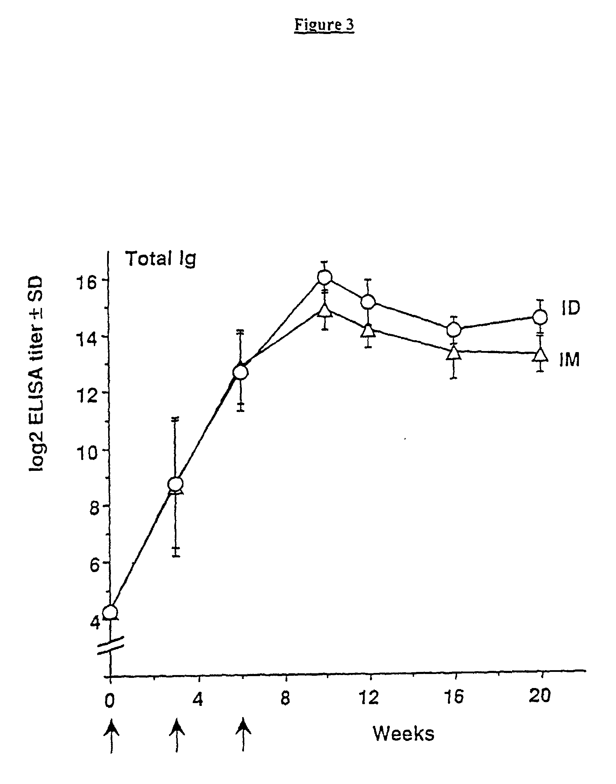

Figure 3 shows the comparison of antibody responses in BALB/c mice after IM or ID jet immunization (Mesoflash™M10) with pV1JHA. Female BALB/c mice (6 per group) were immunized three times (weeks 0, 3 and 6) with 10 µg of pV1JHA plasmid encoding the haemagglutin from A/PR8/34 influenza virus. Immunizations were performed either by injecting the plasmid in 50 µl of 20 mM Hepes buffer pH 7.2 via the IM route into the left quadriceps or by injecting the plasmid in 100 µl of the same buffer via the ID route by using the Mesoflash™M10 injector (see Example 2A). Anti-influenza immunoglobulins were measured on weeks 0 (preimmune), 3, 6, 10, 12, 16 and 20 in the sera of the immunized mice by ELISA titration starting with a 1:25 dilution of the sera. Antibody titers are represented over time as geometric mean endpoint Ig titers on a log2 scale.

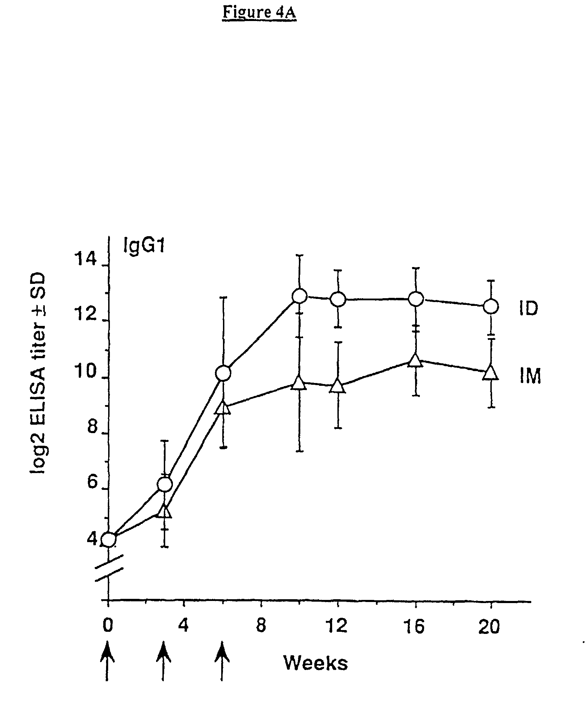

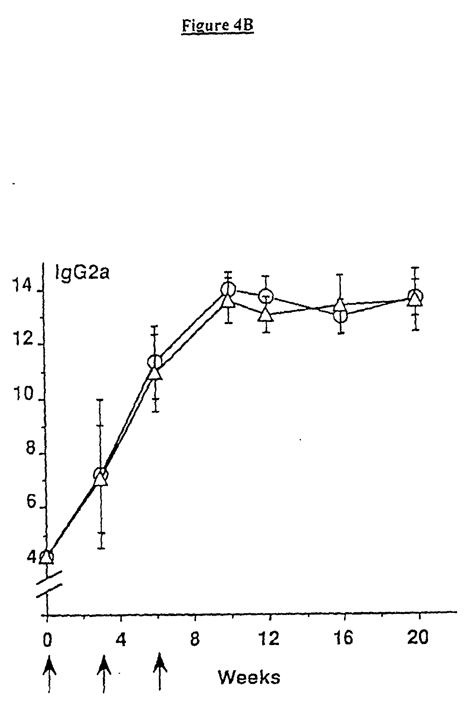

Figures 4A and 4B show the comparison of antibody subtypes in BALB/c mice after IM or ID immunization with pV1JHA. Serum samples were titrated for specific IgG1 and IgG2a isotypes by ELISA against A(PR/8/34 virus (see Example 2A). Antibody titers are represented over time as geometric mean endpoint IgGI (Fig. 4A) and IgG2a (Fig. 4B) titers on a log2 scale.

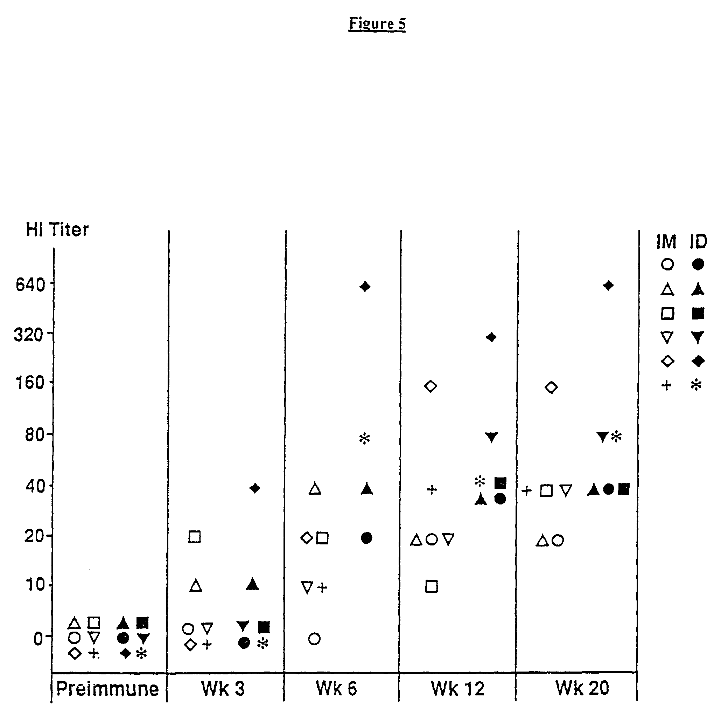

Figure 5 shows the HI antibodies generated by the pV1JHA plasmid DNA vaccine in BALB/c mice. Serum samples collected on weeks 0 (preimmune), 3, 6, 12 and 20 were assayed for HI antibody titers as described in Example 2A starting with a 1:10 dilution of the sera. Endpoint HI titers for each individual animal are represented over time. IM immunized mice are represented with the open symbols, ID immunized mice are represented with the closed symbols.

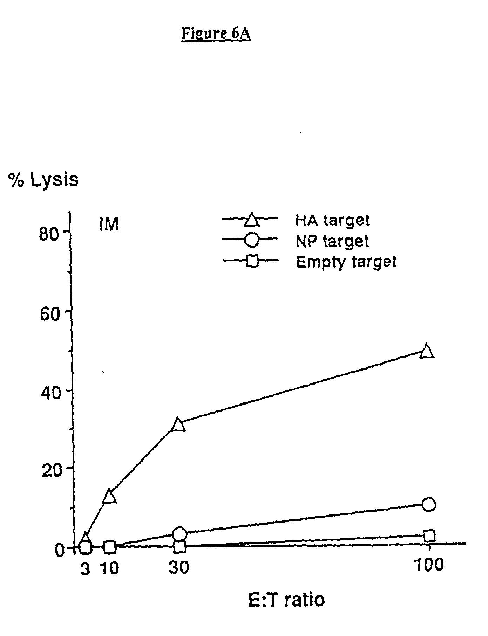

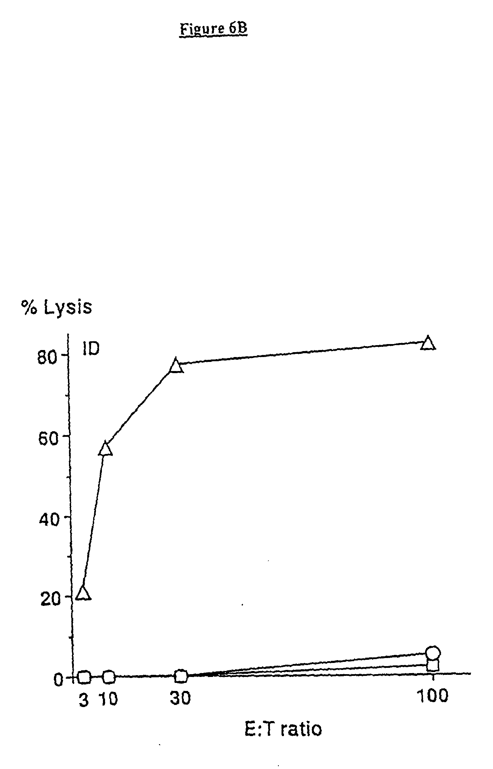

Figures 6A and 6B show the Cytotoxic T-Lymphocyte response generated by the pV1JHA plasmid DNA vaccine in BALB/c mice. At the end (week 20) of the study, mice were sacrificed and spleens were taken to test for the presence of HA-specific CTLs in a standard 4 hours ⁵¹Cr release assay (see Example 2A). P815 target cells were either pulsed with the influenza A/PR/8/34 HA peptide CTL epitope (Δ, "HA target" for the measurement of specific lysis) or NP peptide CTL epitope (○, "NP target" for the measurement of non-specific lysis). Non-pulsed P815 targets (□, "empty targets") were used as an additional negative control in this assay to confirm the absence of non-specific lysis. The percentage of target cell lysis is shown as a function of effector cells to target cells ratio (E:T ratio) for one representative mouse in the IM group (Fig. 6A) for one representative mouse in the ID group (Fig. 6B).

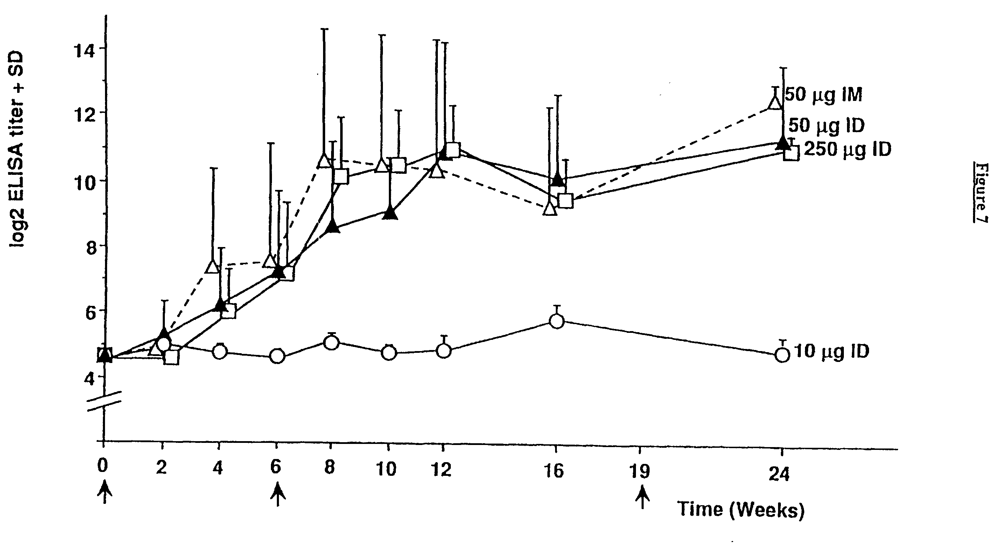

Figure 7 shows the comparison of antibody responses in cynomolgus macaques after IM or ID jet immunization (Mesoflash™M40) with pV1JHA. Cynomolgus macaques (2 males and 2 females per group) were immunized three times (weeks 0, 6 and 19) by the IM route into the quadriceps muscle with 50 µg of pV1JHA plasmid in 500 µl of 9‰ NaCl or by the ID route into the shaved thigh skin with either 10, 50 or 250 µg of pV1JHA plasmid in 100 µl of 9‰ NaCI by using the Mesoflash™M40 injector (see Example 3A). Anti-HA specific antibody titers (total specific immunoglobulins) were followed over time (0-preimmune, 2, 4, 6, 8, 10, 12, 16 and 24 weeks) by ELISA titration as described in Example 2A starting with a 1:25 dilution of the sera. For each immunization regimen (Δ, 50 µg IM; ○, 10 µg ID; Δ, 50 µg ID; □, 250 µg ID) the anti-HA immunoglobulin response is represented over time as geometric mean endpoint titers on a log2 scale.

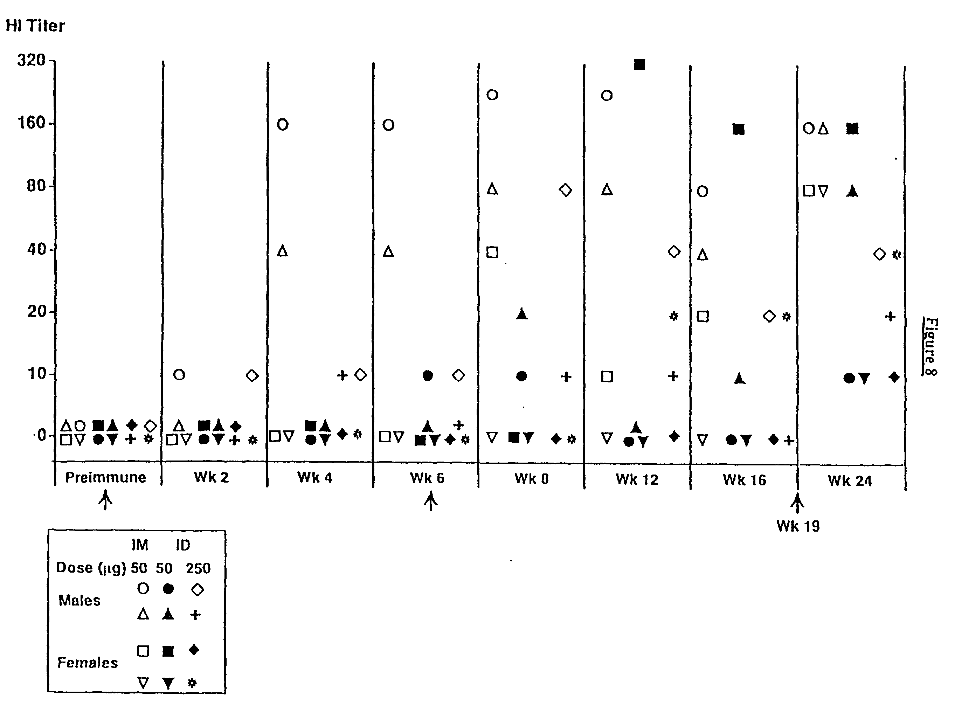

Figure 8 shows the HI antibodies generated by the pV1JHA plasmid DNA vaccine in cynomolgus macaques (Example 3). Serum samples from previous study collected on weeks 0, 2, 4, 6, 8, 12, 16 and 24 were assayed for HI antibody titers as described in Example 2A starting with a 1:10 dilution of the sera. Endpoint HI titers for each individual monkey are represented over time. IM immunized monkeys are represented with ○, Δ, □, ∇ ; other symbols are ID immunized monkeys. Females are represented with □, ■, ∇, ▼, ◆, ∗; other symbols are males.

### Example 1 : p53 vaccination

### 1A - Materials and methods

### Plasmid and virus constructs

The ALVAC recombinant expressing human wild-type p53 (vCP207) has been described in Roth et al, PNAS (1996) 93 : 4781. vP1101, a NYVAC (attenuated vaccinia virus) recombinant expressing human wild-type p53 has been described in Tartaglia et al, Virology (1992) 188 : 217. vCP297 and ALVAC recombinant expressing *Photinus pyralis* luciferase is used as control. Two plasmids expressing human wild-type p53 (pC53-SN₃) and human p53 with an R to H mutation at amino acid 273 (pC53-4.2N₃) were constructed so that the p53 encoding sequences are placed under the control of the human CMV immediate early promoter/enhancer. Both plasmids also contain the neo^{R} marker allowing selection with G418. The plasmid pMP1406hp53, which also expresses p53 under the control of the human CMV immediate early promoter/enhancer, lacks the neo^{R} gene.

### Transformed cell lines

P815 is a murine mastocytoma line of DBA/2 origin. P815 HFR is another isolate of this line. BALB/3T12-3, a tumorigenic fibroblast line derived from BALB/c mouse embryos. was obtained from the ATCC (no. CCL 164).

Cells were transfected with pC53-4.2N₃ using Lipofectamine (GIBCO BRL) for P815 and CaPO₄ for BALB/3T12-3. Transfectants were selected and expanded in the presence of 400µg/ml of G418, and clones that stably expressed p53 were chosen. Clone 2E4 was derived upon transfection from P815, clone 4D5 from P815 HFR and clone 2-21 from BALB/3T12-3.

### Immunization of mice

Eight-week-old BALB/c ByJ (IFFA-CREDO or Jackson Laboratories) or DBA/2 female mice (IFFA-CREDO) were immunized using a syringe for intravenous (100 µl in the tail vein) and subcutaneous or intramuscular administration (100 µl). For direct intrasplenic administration, mice were anesthetized and shaved, and a Mesoflash M10 apparatus (Prolitec) that is adapted for intradermal delivery in mice, was used to deliver 20 µl at each of five separate sites on the back. Solutions to be injected were filled into a 5 ml glass chamber which is lodged in the M10 injector The injector was equipped with a nozzle comprising five orifices and calibrated to deliver fixed volumes of 100 µl per shot. DNA for injection was purified by alkaline lysis followed by CsCI ethidium bromide equilibrium gradient centrifugation or Qiagen column chromatography.

### ELISA for detection of mouse serum antibodies against human p53

The coating antigen was a nuclear extract of Sf9 insect cells infected with a recombinant baculovirus expressing human wild-type p53. The p53 content of this extract was approximately 50 % by electrophoresis. Microtiter plates (96-well) (Dynatech) were coated overnight at 4°C with 100 ng of p53 in 100 µl/well. After washing with PBS-0,005% Tween 20 (PBS-T), plates were blocked for 1 hr at 37°C with PBS-T containing 1% bovine serum albumin (PBS-T-BSA) and washed again. Serum, or as positive control, monoclonal antibody DO1 (Santa Cruz), which recognizes human p53, was added, and plates were incubated for 1.4 hr at 37°C. After washing, the secondary antibody, rabbit anti-mouse IgG, IgA, IgM (Zymed) was added at a dilution of 1/1000 in PBS-T-BSA, and incubation was continued for and additional 1.5 hr at 37°C. After a final wash, the substrate disodium p-nitro-phenyl-phosphate was added at 1mg/ml in 1 M diethanolamine, 0.3 mM MgCl₂, pH 9.8, and plates were developed for 30 min at room temperature in the dark. The reaction was stopped with 50 µl/well 2 N NaOH, and plates were read at 405 nm, using a Molecular Devices plate-reader.

### Determination of specific CTLs against human p53

Spleens from immunized mice were dissociated in RPMI with 2% fetal calf serum (FCS), and splenocytes were prepared. Stimulators (from unimmunized mice : prepared as above) were infected at a multiplicity of infection 2-10 with vP1101 or vCP207, as indicated.

After 1 hr at 37°C with 5% CO₂, the infected stimulators were washed and irradiated with 25 Gy (or in some experiments were used without irradiation) and mixed with effectors at a 1:2 or 1:5 ratio in a 75 cm³ flask at a final concentration of 2 x 10⁶ cells/ml. Flasks were incubated vertically for 5 days in a 37°C incubator with 5% CO₂. If a second stimulation was performed, a stimulator : effector ratio of 2:1 was used, and 10 U/ml murine interleukin-2 (IL-2 ; Genzyme) were added. The restimulation was done for 5 days.

For the CTL assay, 10⁶ P815 or 2E4 cells were labelled with 200 µCi of NA₂⁵¹CrO₄ (NEN, 40 mCi/ml) for 1 hr at 37°C and washed three times in RPMI with 10% FCS. 5 x 10³-10⁴ target cells were dispensed into each well of a 96-well round-bottom microtiter dish. Effectors were added, and plates were incubated for 4 hrs at 37°, after which the supernatant was harvested and counted. Percentage specific lysis was calculated as 100 x (effector release-spontaneous release) / (total release-spontaneous release).

### 1B - Results

### Preliminary experiments using a canary pox (ALVAC vector encoding human wild-type p53 (vCP207))

The immune response (antibody and CTL responses) to vCP207 delivered by different routes was studied and results are as follows.

Intravenous administration at weeks 0, 3 and 5 of 107 TCID₅₀ of the recombinant, but not the parental ALVAC, induced a significant specific IgG response in 11/12 mice. However, when the same vaccination was performed by the subcutaneous route, only three out of 12 mice responded, and even these had titers only marginally above background. We also saw very little response by the intradermal route.

We also examined the specific CTL activity of splenocytes from immunized mice after *in vitro* stimulation with naive splenocytes infected with a recombinant highly attenuated vaccinia, NYVAC, expressing human wild-type p53 (vP1101). Splenocytes from mice immunized three times intravenously with vCP207 showed high levels of lysis of clone 2E4, a P815 line transfected with human R273H mutant p53. There was clear lytic activity, even after one round of *in vitro* stimulation, although the levels increased, especially at low E:T ratios, after a second round *in vitro* stimulation. The response was specific for p53, since we saw only low levels of lysis of 2E4 after immunization with the ALVAC vector and only low levels of lysis of untransfected P815. In contrast to the results by the intravenous route, splenocytes from mice immunized subcutaneously with same vCP207 showed no evidence of CTL activity, even after two rounds of *in vitro* stimulation. Intradermal administration also failed to elicit any specific anti-p53 CTLs.

Finally, we considered that the route of administration might be critical only for priming ; however, mice primed by the intravenous route and boosted subcutaneously failed to respond.

We concluded that the intravenous route was the optimal route to induce an immune response specific for p53, when using a recombinant canary pox as immunogen.

### Immune response (antibody and CTL responses) to non-infectious DNA encoding human wild-type p53

We have also examined the response to immunization with a plasmid encoding human wild-type p53 under the control of the CMV promoter (pC53-SN₃).

Intradermal administration of 100 µg of this plasmid at weeks 0, 3 and 6 induced a specific antibody response in three out of eight mice, compared to none of the mice for the pUCneo vector. Despite this rather variable antibody response, we saw a CTL response in all groups of animals immunized with 100 µg, but not 1 or 10 µg of pC53-SN₃ (Figure 1A). Like those induced by vCP207, the CTLs induced by p53 DNA were specific for p53, showing no lysis of 2E4 following immunization with the pUCneo vector DNA, nor any significant lysis of untransfected P815. We also saw a clear CTL response after intramuscular injection of 100 µg of another DNA encoding human wild-type p53, pMP1406hp53 (Figure 1B).

### Protection against challenge with human p53-transfected cell lines after immunization with vCP207 or pc53-SN₃

Once we had determined optimal ways to induce anti-p53 immune responses, we tested whether they would be protective against challenge with murine tumor lines transfected with human p53. In these experiments, we immunized at weeks 0, 3 and 6 and challenged at week 8. One challenge line, clone 2-21, was derived by transfecting BALB/3T12-3, a tumorigenic isolate of BALB/c 3T3, with a plasmid encoding human mutant p53 (R273H). Control immunized BALB/c mice developed tumors, which grew progressively, starting about 6 weeks after subcutaneous injection of 10⁷ cells (Figure 2A). Prior intravenous immunization with vCP207 conferred partial, but statistically non-significant (P > 0.25 vs. PBS) protection, with only one out of nine mice remaining tumor-free by week 15. This effect was specific for p53, since immunization with the ALVAC vector was not protective, and, in fact, appeared to slightly accelerate tumor growth relative to PBS. Also, vCP207 conferred no protection at all against the untransfected parent BALB/ET12-3 (Figure 2B). By contrast to the partial protection induced by the recombinant virus, intradermal immunization with pC53-SN₃ DNA induced complete protection (P < 0.001 vs. both PBS and pUCneo ; Figure 2C).

Another challenge model, clone 4D5, which was derived by transfecting P815 mastocytoma cells with human R273H mutant p53, forms tumors that appear about 3 weeks after subcutaneous injection of 10⁴ cells in DBA2 mice (Figure 2D) and grow rapidly, resulting in death by about week 6. In this model, both vCP207 and pC53-SN₃ induced partial protection (Figure 2D), but only the DNA was significantly different from the PBS control group (P < 0.1 for vCP207 and p < 0.01 for pC53-SN₃). Neither the control ALVAC nor the control DNA induced a significant protection relative to PBS (P < 0.25).

### 1C - Discussion

We have compared the immune responses induced by delivering the full-length wild-type p53 gene, either *via* a recombinant viral vector or plasmid DNA. In the case of the recombinant ALVAC, the route of administration was critical for the induction of an optimal immune response. We saw good responses immunizing by the intravenous route, as others have reported for fowlpox. However, the antibody response was significantly weaker by the subcutaneous, intramuscular and intradermal routes did not induce any detectable CTLs.

While the levels of CTLs generated by immunization with DNA and recombinant poxvirus appeared similar, it is difficult to compare the doses used because of the different nature of the two vectors. Nevertheless, whereas 100 µg of DNA and 10⁷ virus were sufficient to induce CTLs, in both cases, a 10-fold lower dose failed to induce CTLs ; *i.e.* neither 10 µg of DNA (Figure 1A), nor 10⁶ vCP207 reproducibly induced detectable CTLs. It is also worth bearing in mind that DNA induced CTLs when given intradermally or intramuscularly, which are inherently more practical and less likely to raise safety concerns than the intravenous route used with the recombinant virus.

Despite generating CTL, intravenous immunization with vCP207 provided only partial protection in the transfected 3T3 and P815 models.

In brief, the immune response and protection data with human p53 can be summarized as follows.

| **Immunization** | **Route** | **Abs** | **CTLs** | **Protection** |
|---|---|---|---|---|
| vCP207 | i.v | ++ | + | +/- |
| ALVAC (control) | i.v. | - | - | - |
| phup53 | i.m. | +/- | + | - |
| pvec (control) | i.m. | - | - | - |
| phup53 | i.d.(Mesoflash) | + | + | + |
| pvec (control) | i.d. (Mesoflash) | - | - | - |

It is immediately apparent that superior results were obtained upon DNA immunization by the intradermal route, using a needle-free injector.

### Example 2 : flu vaccination in mice

### 2.A - Materials and methods

### Plasmids

The pV1JHA plasmid is a pUC19-derived plasmid contains ampicilline resistance gene marker, the haemagglutinin (HA) gene cloned from the A/PR/8/34 influenza virus, the enhancer, promoter and intron A from the hCMV IE1 gene and the poly(A) signal sequence from the bovine growth hormone gene (Montgomery et al, (1993) 12 : 777).

This plasmid was grown in *E. coli* XL1-blue strain, extracted by the standard alcaline lysis procedure followed by selective precipitation of RNAs by LiCl/ethanol. The supernatant was then sequentially digested with RNaseA ( 0.01-0.05 mg per mg of DNA) and proteinase K (0.04-0.2 mg per mg of DNA). The resulting DNA was phenol/chloroform extracted. Plasmid DNA was further purified on a CsCl-EtBr gradient. The EtBr was removed by 1-butanol extraction. The plasmid was EtOH-precipitated, resuspended in distilled water and stored at a concentration of 5 mg/ml at -20°C. The concentration and purity of the plasmid preparations were determined by agarose gel electrophoresis and by OD 260/280 readings. The OD 260/280 ratios were ∼ 1.8.

### Immunization of mice

Eight-week old female BALB/c mice (Charles River Laboratories, Saint Aubin les Elbeuf, France) were anaesthetized by IP injection of a solution containing 8mg/ml ketamine (Imalgène 500, Rhône-Mérieux) and 1.6 mg/ml xylazine (Rompun 2%, Bayer) in a total volume of 0.2 ml of saline. Female BALB/c mice (6 per group) were immunized three times (weeks 0, 3 and 6) with 10 µg of plasmid pV1JHA. Immunizations were performed as follows ;

For IM injections, 50 µl of DNA in 9‰ NaCl was injected into the quadriceps muscle of a shaved thigh swabbed with ethanol by using a Micro-Fine insuline syringe equipped with a 29G1/2 needle.

For ID injections, 100 µl of DNA in 9‰ NaCl was injected into five spots of shaved dorsal skin swabbed with ethanol by using a Mesoflash™ M10 jet injector (Mesoflash™series, Prolitec, Aouste/Sye, France). Solutions to be injected were filled into the 5 ml glass chamber of the M10 injector. This injector was equipped with a rectangular nozzle comprising five orifices and calibrated to deliver fixed volumes of Ca.100 µl per shot.

Serum samples were recovered on weeks 0 (preimmune), 3, 6, 10, 12, 16 and 20. Anti-influenza immunoglobulins were measured by ELISA titration starting with a 1:25 dilution of the sera. Sera were titrated for specific IgG1 and IgG2a isotypes by ELISA against A/PR/8/34 virus and assayed for HI antibody titers, starting with a 1:10 dilution of the sera. On week 20, mice were sacrificed and spleens were taken to test for the presence of HA-specific CTLs in a standard 4 hours ⁵¹Cr release assay.

### ELISA assay

All volumes used in this ELISA were 100 µl/well, unless stated otherwise.
Maxysorp 96-well plates (Nunc) were coated overnight at 4°C with whole influenza virus (A/PR/8/34) at 100 ng/100 µl (total protein) in 0.1 M carbonate-bicarbonate buffer, pH 9.6. Plates were washed two times with PBS containing 0.05% v/v Tween 20 (washing solution) and blocked with PBS containing 0.05% v/v Tween 20 and 1% w/v non-fat milk (blocking solution). After 1 hr incubation at 37°C with gentle shaking, plates were washed again twice. Then the following reagents were sequentially transferred to the plates and incubated for 1.5 hr at 37°C with gentle shaking and extensive washing (4 cycles) between each incubation: i) serum samples diluted from 1/25-1/51200 by 2 fold serial dilutions in the blocking solution, ii) biotinylated sheep anti-mouse IgG (respectively sheep antihuman, in the monkey ELISA) (Amersham) diluted to 1/5000 in the blocking solution, ii) streptavidine-biotinylated horseradish peroxidase complex (Amersham) diluted to 1/2000 in the blocking solution.

To detect specific immunoglobulin isotypes in the mouse ELISA, biotinylated sheep anti-mouse IgG1 and IgG2a were used.

For color development, 0.1 M sodium citrate buffer pH 5.6 containing 0.03% hydrogen peroxide and 1 mg/ml O-phenylenediaminedihydrochloride (Sigma) was added. The colour reaction was stopped after 30 min by addition of 50 µl of 2M H2SO4 and absorbances were read at 490 nm using a vertical beam spectrophotometer (Titertek, Flow Laboratories). Antibody titers were defined as the reciprocal serum dilution with an A490 value of 0.5. Mean serum antibody titers were expressed as geometric mean titers (GMT) on a log2 scale.

### Haemagglutination inhibition (HI) assay

Prior to the assay, sera were cleared from non specific inhibitors with Receptor Destroying Enzyme (RDE, Sigma) and from cold agglutinins by hemadsorption. In brief, sera (25 µl) were incubated overnight at 37°C with 125 µl of RDE (Freeze-dried powder from 5 ml of Vibrio culture fluid reconstituted with 2.5 ml of distilled water) and heated for 1 hr at 56°C. The treated samples (150 µl) were then preabsorbed for 30 min at room temperature with 125 µl of chicken red blood cells at a concentration of 10% (w/v) in PBS and the cold-agglutinins removed with the cells by 10 min centrifugation at 3000 rpm. These sequential treatments induced a ca. 10 fold dilution of sera.

Two fold serial dilutions of sera (from 1/10 to 1/10240 in PBS) were then prepared in 96-well plates (Greiner-Labor-Tecknik, 50 µl/well) and 50 µl of PBS containing 4 HA units of influenza virus were added to each well and the plates incubated for 1 hr at room temperature. Fifty µl of chicken red blood cells at a concentration of 0.5% (w/v) in PBS were added for 1 hr and agglutination was determined by visual examination. HI titer was expressed as the reciprocal of the highest dilution of the serum inducing haemagglutination inhibition.

### Cytotoxicity assay.

Splenocytes from immunized mice (effectors) were isolated and restimulated in vitro by co-culture for 7 days with syngeneic spleen cells (stimulators) that had been infected with A/Singapore/6/86 influenza virus (H1N1 strain, 100 HA units / 10⁶ splenocytes) and for 4 additional days with fresh, influenza-infected syngeneic spleen cells that had been treated with mitomycin (2.5 µg / 10⁶ splenocytes). Approximately 7.5x10⁷ effectors and stimulators were placed together in flasks in 30 ml of RPMI 1640 medium containing 10% FCS, 50 µM 2-mercaptoethanol, glutamine (2mM) and antibiotics (=culture medium). For the first stimulation the effector/stimulator ratio was 3:2 and for the second stimulation the effector/stimulator ratio was 1:4 and murin recombinant IL-2 (Boehringer Mannheim) was added to the culture medium at 10 units/ml.

Cytotoxic activities were measured in a standard 4-hr ⁵¹Cr-release assay. In brief, the stimulated effector cells were washed by centrifugation and diluted in 96-well plates (Falcon) from 500 000 cells/100 µl to 18500 cells/100µl by 3 fold serial dilution. P815 (H-2K^{d}) mastocytoma cells (targets) were sensitized by incubation with a peptide comprising the A/PR/8/34 HA2 CTL epitope in the H-2K^{d} haplotype (13) (IYSTVASSLVL, 20 µg / 10⁶cells) or with the A/PR/8/34 NP CTL epitope (14) (TYQRTRALVTG, 20 µg / 10⁶cells = negative control) and loaded with 51Cr (300 µCi / 10⁶ cells). Five thousand target cells in 100 µl of culture medium were added to the effector cells in the 96 well plates. After 4 hrs at 37°C, the plates were centrifuged at 1400 rpm for 3-4 min and 30 µl of the supernatants from each well were transferred into the corresponding well of a 96-well Lumaplate (Packard) for radioactivity counting using a TopCount beta-counter (Packard).

Results are expressed as percentage of specific ⁵¹Cr release, calculated by using the formula: (a-c/b-c)x100, where a=cpm released in the presence of effector cells, b=cpm released in the presence of detergent (maximal release) and c=cpm released in the control wells without effector cells (spontaneous release).

### 2B - Results

The immune responses induced by ID jet-immunization were compared to that of standard IM injection in the influenza model with pV1JHA encoding the haemagglutinin of influenza A/PR/8/34 virus (H1N1 strain). In BALB/c mice, a dose-response relationship was evident between the dose of pV1JHA plasmid and anti-influenza antibodies generated after IM or ID jet injection (Mesoflash™M10) of pV1JHA, the lowest immunogenic dose being 0.1 µg in a two immunizations protocol (not shown). In this study, aiming to compare the two routes of administration in a protocol comprising 3 immunizations with 10 µg of pV1JHA (3 weeks apart), the antibody production (total immunoglobulins) after jet injection of the DNA vaccine to the skin was slightly superior (1-2 ELISA dilutions, 2-4 fold) to that induced by IM immunization (Figure 3).

Considering the low level of plasmid expression in the skin, this immunization experiment suggests that potent immune responses can be generated from very little protein immunogen if this is produced or taken up in appropriate cells or tissues.

When we examined the IgG1/IgG2a balance, it appeared that only the IgG1 titers were affected by the route of immunization. Indeed, Figure 4 shows that IM and ID jet immunized mice had very similar IgG2a titers whereas the mean IgG1 titer in the ID jet immunized mice reached a maximum plateau value which was about 8 fold (3 ELISA dilutions) above the mean titer observed in the IM group. The higher IgGI titers obtained by ID jet injection correlated positively with higher virus neutralizing titers as measured by standard haemagglutination inhibition assay on fresh chicken erythrocytes incubated with A/PR8/34 Influenza virus. HI titers were slightly higher in the ID jet immunized group as compared to the IM group (Figure 5).

In addition to HI titers, the DNA vaccine induced also anti-HA specific CTL responses in the immunized BALB/c mice. We detected significant CTL responses five months after the primary immunization in 4 out of the 6 IM immunized mice and in 3 out of the 5 jet immunized mice that were tested. Figure 6 shows the specific CTL responses generated in one representative responder from each of the two groups.

### Example 3 : Flu vaccination in monkeys

### 3A - Material and methods

**Plasmid** is as described in 2A

### Immunization of monkeys

Two year old male and female Cynomolgus macaques (*Macaca fascicularis*, 2,1-3.3 kg at the time of the primary immunisation) originating from Vietnam were obtained from Naforimex (Hochiminh Ville) and were tuberculinated and quarantined immediately after arrival in our animal care facilities. Before entering this study, the monkeys had received three injections of inactivated polio vaccine (Pasteur Mérieux Connaught). Monkeys were housed according to the guidelines of European regulations and provided with tap water ad libitum, fruits and commercial primate chow.

Monkeys were anaesthetized by IM administration in the thigh of 50 mg of ketamine (0.5 ml of Imalgene 1000, Rhône-Mérieux). The injection site was shaven and swabbed with ethanol prior to the injection. Cynomolgus macaques (2 males and 2 females per group) were immunized three times (weeks 0, 6 and 19) as follows :

For IM injections, 50 µg of plasmid pV1JHA in 500 µl of 9‰ NaCl was injected into the quadriceps muscle by using a 3 ml syringe equipped with a 26G3/8 needle.

For ID injections, 10, 50 or 250 µg of plasmid pV1JHA in 100 µl of 9‰ NaCI were injected into five spots of thigh skin by using a Mesoflash™ M40 jet gun (Mesoflash™series, Prolitec, Aouste/Sye, France). Solutions to be injected were filled into a syringe which adapts onto the M40 injector. This injector was equipped with a circular nozzle comprising five orifices and calibrated to deliver fixed volumes of Ca.100 µl per shot.

Serum samples were recovered on weeks 0 (preimmune), 2, 4, 6. 8, 10, 12, 16 and 24. Anti-HA specific antibody titers (total specific immunoglobulins) were followed over time by ELISA titration as described in 2A, starting with a 1:25 dilution of the sera. Serum samples were also assayed for HI antibody titers as described in 2A, starting with a 1 : 10 dilution of the sera.

### 3B - Results

We compared ID jet-gun immunization (Mesoflash™M40) to IM immunization in monkeys. For jet-gun immunization we performed a dose-response type of study and again observed a correlation between the amount of pV1JHA used for the immunization and the magnitude of the subsequent antibody response. We found that the 50 µg dose of pV1JHA induced stronger ELISA and HI titers than the 10 µg dose which did not evoke any response. The magnitude of the antibody response could not be improved by increasing the dose of pV1JHA from 50 µg to 250 µg but the responses appeared more homogenous in the 250 µg group.

The comparison between IM and ID jet-gun immunization with 50 µg of pV1JHA revealed no significant difference in the overall antibody response. However, the examination of individual titers showed a faster seroconversion in two of the IM monkeys. In both groups the responses were improved by the booster immunization given 3 months after the second administration.

## Claims

1. The use of a solution comprising a non-infectious DNA molecule able to express in a vertebrate, an antigen that is (a) specific for a micro-organism able to induce an infection in a vertebrate or (b) tumour-associated, in the preparation of a medicament for treating or preventing an infection or a tumoral disorder, that is intended to be exclusively delivered to the vertebrate skin so that a balanced Th1/Th2 immune response is induced against the antigen ; the delivery of said medicament being achieved using a needle-free apparatus having pressure means to propel the medicament, the pressure strength of which is adjusted as to deliver the medicament exclusively to the skin.

2. The use according to claim 1, wherein the medicament is intended to be exclusively delivered to the epidermal layer of the skin.

3. The use according to claim 1, wherein the medicament is intended to be exclusively delivered to the dermal layer of the skin.

4. The use according to any one of claims 1 to 3, wherein the DNA molecule is capable of expressing the tumor-associated p53 antigen.

5. The use of claim 4, wherein the p53 antigen is of human origin.

6. The use of claim 4 or 5, wherein the p53 antigen is a wild-type p53.

7. The use of claim 4 or 5, wherein the p53 antigen is a mutant p53.

## Patentansprüche

1. Verwendung einer ein nichtinfektiöses DNA-Molekül, das in einem Vertebraten ein Antigen, das (a) spezifisch für einen zur Induktion einer Infektion in einem Vertebraten fähigen Mikroorganismus oder (b) tumorassoziiert ist, exprimieren kann, enthaltenden Lösung bei der Herstellung eines Arzneimittels zur Behandlung einer Infektion oder Tumorerkrankung oder zur Vorbeugung dagegen, das ausschließlich an die Haut des Vertebraten abgegeben werden soll, so daß eine ausgewogene Th1/Th2-Immunantwort gegen das Antigen induziert wird; wobei die Abgabe des Arzneimittels durch Verwendung eines nadelfreien Geräts mit einer Druckvorrichtung zum Hineintreiben des Arzneimittels, deren Druck so eingestellt wird, daß das Arzneimittel ausschließlich an die Haut abgegeben wird.

2. Verwendung nach Anspruch 1, wobei das Arzneimittel ausschließlich an die Epidermisschicht der Haut abgegeben wird.

3. Verwendung nach Anspruch 1, wobei das Arzneimittel ausschließlich an die Dermisschicht der Haut abgegeben wird.

4. Verwendung nach einem der Ansprüche 1 bis 3, wobei das DNA-Molekül das tumorassoziierte p53-Antigen exprimieren kann.

5. Verwendung nach Anspruch 4, wobei das p53-Antigen menschlichen Ursprungs ist.

6. Verwendung nach Anspruch 4 oder 5, wobei es sich bei dem p53-Antigen um einen p53-Wildtyp handelt.

7. Verwendung nach Anspruch 4 oder 5, wobei es sich bei dem p53-Antigen um eine p53-Mutante handelt.

## Revendications

1. Utilisation d'une solution comprenant une molécule d'ADN non-infectieuse capable d'exprimer chez un vertébré, un antigène qui est (a) spécifique d'un micro-organisme capable d'induire une infection chez un vertébré ou (b) associé à une tumeur, dans la préparation d'un médicament destiné au traitement ou à la prévention d'une infection ou d'une affection tumorale, qui est p révu pour être délivré exclusivement dans la peau du vertébré afin d'induire à l'encontre de l'antigène une réponse immune Th1/Th2 équilibrée ; la délivrance dudit médicament étant mise en oeuvre en utilisant un appareil sans aiguille incorporant des moyens de pression permettant de propulser le médicament et dont la force de pression est ajustée afin de délivrer le médicament exclusivement dans la peau.

2. Utilisation selon la revendication 1, et selon laquelle le médicament est prévu pour être exclusivement délivré dans la couche épidermique de la peau.

3. Utilisation selon la revendication 1, et selon laquelle le médicament est prévu pour être exclusivement délivré dans la couche dermique de la peau.

4. Utilisation selon l'une des revendications 1 à 3, et selon laquelle la molécule d'ADN est capable d'exprimer l'antigène p53 associé à une tumeur.

5. Utilisation selon la revendication 4, et selon laquelle l'antigène p53 est d'origine humaine.

6. Utilisation selon la revendication 4 ou 5, et selon laquelle l'antigène p53 est de type sauvage.

7. Utilisation selon la revendication 4 ou 5, et selon laquelle l'antigène p53 est un mutant p53.
